# EUROPEAN PATENT SPECIFICATION

(11) **EP 2 883 052 B1**
(45) Date of publication and mention of the grant of the patent: **26.09.2018**
(21) Application number: 13753421.0
(22) Date of filing: 12.08.2013
(51) Int. Cl.: G01N 33/564, C07K 14/47

(54) **BIOMARKERS**
BIOMARKER
BIOMARQUEURS

(30) Priority: 10.08.2012 US 201261681638 P
(43) Date of publication of application: 17.06.2015
(73) Proprietor: Instituto Superiore Di Sanita', 00161 Roma (IT)
(72) Inventor: LAVIOLA, Giovanni, I-00161 Roma (IT); ADRIANI, Walter, I-00161 Roma (IT); PORFIRIO, Maria Cristina, Roma (IT); CURATOLO, Paolo, Roma (IT); GRANSTREM, Oleg, St. Petersburg 194100 (RU)
(74) Representative: Predazzi, Valentina
(86) International application number: PCT/EP2013/066845
(87) International publication number: WO 2014/023852

(56) References cited:
- W Adriani ET AL: "Blood titers of auto-antibodies to DAT as biomarkers for the risk of alcoholism: The case of positive familiarity (P13)" In: "Needs and Challenges in Translational Medicine: filling the gap between basic research and clinical applications; Abstract book of the international meeting at the Istituto Superiore di Sanita, Rome, Italy, October 1-3, 2008", 1 October 2008 (2008-10-01), XP055084418, page 90, the whole document, in particular l. 14-16 of abstract
- A. M. ANDRES ET AL: "Heterogeneous Rate of Protein Evolution in Serotonin Genes", MOLECULAR BIOLOGY AND EVOLUTION, vol. 24, no. 12, 29 June 2007 (2007-06-29) , pages 2707-2715, XP055084424, ISSN: 0737-4038, DOI: 10.1093/molbev/msm202 -& DATABASE UniProt [Online] 1 October 2000 (2000-10-01), "RecName: Full=Transporter; Flags: Fragment;", XP002715108, retrieved from EBI accession no. UNIPROT:Q9NYN7 Database accession no. Q9NYN7
- UJJWAL K ROUT ET AL: "Presence of GAD65 autoantibodies in the serum of children with autism or ADHD", EUROPEAN CHILD & ADOLESCENT PSYCHIATRY, STEINKOPFF-VERLAG, DA, vol. 21, no. 3, 10 February 2012 (2012-02-10), pages 141-147, XP035026171, ISSN: 1435-165X, DOI: 10.1007/S00787-012-0245-1
- Giovanni Laviola ET AL: "Implication of autoantibodies to receptor fragments in etiology of compulsive behavior and drug addiction" In: "Istituto Superiore di Sanita: ISS-NIH Collaborative Programme: final report of the projects", 1 May 2009 (2009-05-01), XP055084435, pages 374-377, the whole document, in particular section "state of the art" spanning p. 374 and 375
- MACRI S ET AL: "Resilience and vulnerability are dose-dependently related to neonatal stressors in mice", HORMONES AND BEHAVIOR, ACADEMIC PRESS, NEW YORK, NY, US, vol. 56, no. 4, 1 October 2009 (2009-10-01), pages 391-398, XP026683035, ISSN: 0018-506X, DOI: 10.1016/J.YHBEH.2009.07.006 [retrieved on 2009-07-24]
- VANDENBERGH D J ET AL: "A human dopamine transporter cDNA predicts reduced glycosylation, displays a novel repetitive element and provides racially-dimorphic TaqI RFLPs", MOLECULAR BRAIN RESEARCH, ELSEVIER SCIENCE BV, AMSTERDAM, NL, vol. 15, no. 1-2, 1 September 1992 (1992-09-01), pages 161-166, XP024331783, ISSN: 0169-328X, DOI: 10.1016/0169-328X(92)90165-8 [retrieved on 1992-09-01] -& DATABASE UniProt [Online] 1 April 1993 (1993-04-01), "RecName: Full=Sodium-dependent dopamine transporter; Short=DA transporter; Short=DAT; AltName: Full=Solute carrier family 6 member 3;", XP002715109, retrieved from EBI accession no. UNIPROT:Q01959 Database accession no. Q01959

## Description

### FIELD OF THE INVENTION

The present invention relates to methods of diagnosing and monitoring the efficacy of treatment in patients with attention deficit hyperactivity disorder.

### BACKGROUND OF THE INVENTION

Attention deficit hyperactivity disorder (ADHD) affects about 4% of children and may persist into adulthood (Biederman, 1998). Although the mechanisms of ADHD are not understood, the central role of dopamine and norepinephrine in promoting psychomotor stimulation implicates brain catecholamine systems in ADHD (Seeman and Madras, 1998). One focus of ADHD research has been the dopamine transporter (DAT) in brain.

Existing approaches for ADHD functional diagnostics include PET and fMRI neuroimaging. The clinical diagnosis of ADHD is currently based on structured interviews or a structured questionnaire. Uncertainty of the criteria used for ADHD diagnostics has resulted in an exponential rise in prescribed stimulants. The side effects of these drugs are well known.

Development of a diagnostic test for ADHD has been hampered by the elusive nature of the underlying pathophysiology (Madras *et al*., 2000).

Some few biochemical correlations are currently known. Abnormal levels of the dopamine transporter were detected in the brains of subjects with ADHD. Density of the dopamine transporter was measured by single photon emission computed tomography (SPECT) with the highly selective radio-pharmaceutical iodine¹²³-labelled altropane (Fischman *et al*., 1998). The density of dopamine transporter was approximately 70% higher in patients with ADHD (Dougherty *et al.,* 1999). Dopamine transporter binding potential for patients with ADHD exceeded the mean values of the control group by two standard deviations. As the transporter functions principally to reduce synaptic dopamine levels, over-expression of the protein may lead to compensatory increases in dopamine release. Stimulant drugs such as methylphenidate and pemoline may normalise resting dopamine levels and reduce evoked release of dopamine (Seeman and Madras 1998). Thus, it appears that DAT is of significance in ADHD pathogenesis.

It has recently been demonstrated that changes in brain receptor expression, degradation ratio and immunoreactivity may result in detectable outcomes in parts of the body other than the CNS. Specific autoimmune biomarkers have now been found for opiate-induced addiction, epilepsy and brain ischemia (Granstrem *et al.,* 2006; Dambinova *et al.,* 1998; Dambinova *et al.,* 2003). Autoantibodies (aAbs) to mu-delta recombinant receptor (MDOR) fragment were revealed in mice as a result of chronic morphine administration (Granstrem *et al.,* 2006). Previously, receptor autoradiography was able to demonstrate an increased density of mu-opiate receptors in the hippocampus of morphine-exposed rats (Werling *et al*, 1989). High levels of aAbs to MDOR were also observed in the blood of human addicts. This elevation of MDOR aAbs levels corresponded to severity of opiate intake, suggesting that MDOR may be a hallmark of opiate addiction (Dambinova *et al*., 2003).

Similarly, accumulation of AMPA GluR1 aAbs was found to correlate with spiking of brain activity, as defined by EEG, and preceded the manifestation of seizures induced by cobalt implantation in rats (Dambinova *et al.,* 1998). Human epileptic patients demonstrated significantly higher levels of GluR1 aAbs in comparison with healthy donors (Granstrem *et al.,* 2001). Also, aAbs to NMDA glutamate receptors were found in the blood of patients with transient ischemic attack and stroke (Dambinova *et al.,* 2003). This finding was confirmed in rats prepared according to experimental brain ischemia models (Gappoeva *et al.,* 2003).

There have been previous attempts to use the properties of DAT as biomarkers for diagnostics and prediction of certain vulnerable constitutions. For example, WO 2005/118848 discloses methods to predict the likelihood of suicidal or self-destructive behaviour in a patient during treatment. The method employs the detection of a VNTR polymorphism in the 3'-UTR of the dopamine transporter gene (SLC6A3). Patients with nine or fewer repeats in the polymorphic region are considered poor responders to clozapine, with poor expression of the SLC6A3 gene. Also disclosed are methods of treatment which depend on the presence or absence of this polymorphism or surrogate markers thereof, and kits for use therewith.

WO 2005/106038 discloses biomarkers that are diagnostic of nerve cell injury and/or neuronal disorders, as well as being diagnostic of the degree of severity of nerve injury, the cell(s) involved in the injury, and the subcellular localisation of the injury.

Adriani et al International Meeting "Needs and Challenges in Translational Medicine: filling the gap between basic research and clinical applications" Istituto Superiore di Sanità Rome, Italy October 1-3, 2008 "Blood titers of auto-antibodies to dat as biomarkers for the risk of alcoholism: the case of positive familiarity" discloses an ELISA screen based on a DAT-Asp epitope.

Rout et al, Eur Child Adolesc Psychiatry (2012) 21:141-147, "Presence of GAD65 autoantibodies in the serum of children with autism or ADHD" discloses the detection of GAD65 autoantibodies in serum of ADHD affected autistic children and the concept that serum anti-GAD65 antibodies may be a common marker of subgroups of patients with autism and ADHD.

Laviola et al, May 2009, pages 374-377, "Implication of autoantibodies to receptor fragments in etiology of compulsive behavior and drug addiction" in: "Istituto Superiore di Sanita: ISS-NIH Collaborative Programme: final report of the projects" discloses that autoantibodies against DAT are involved in the etiology of compulsive behaviour and drug addiction in mice.

Macri et al, Hormones and Behavior (2009) 56(4):391-398, "Resilience and vulnerability are dose-dependently related to neonatal stressors in mice" discloses that autoantibodies against DAT are involved in neonatal stress in mice.

### OBJECTS OF THE INVENTION

1) It is an object of the present invention to provide methods and biochemical markers for distinguishing between neuropsychiatric symptoms that may be due to a metabolic imbalance in the brain, whereby the genetic over-expression of human dopamine transporter (hDAT) and its subsequent catabolism may trigger auto-immune responses in the periphery related to the trafficking of hDAT and/or its catabolites in the bloodstream.
2) It is another object of the invention to provide methods and biochemical markers for diagnosing ADHD in humans, and for distinguishing between ADHD and similar neuropsychiatric syndromes not caused by intrinsic genetic and/or biochemical impairments of dopaminergic system.
3) It is another object of the invention to provide methods and biochemical markers for monitoring of efficacy of treatment in patient with ADHD diagnosis.

### SUMMARY OF THE INVENTION

It has now been found, surprisingly, that naturally-occurring auto-antibodies (aAb) to fragments of hDAT can be found in the blood and other biological fluids of humans with ADHD.

Accordingly, in a first aspect, the present invention provides a method for assisting in the diagnosis of attention deficit hyperactivity disorder in a subject, comprising assaying a sample of a biological fluid obtained from the subject for the presence of antibodies capable of recognising at least one peptide selected from those characterised by SEQ ID NO's 2, 4, 5.

In an alternative aspect, there is provided a method for determining the likelihood a subject suspected of having ADHD comprising determining whether a sample of biological fluid obtained from said subject contains a statistically significant level of antibodies capable of recognising at least one peptide selected from those characterised by SEQ ID NO's 2, 4, 5.

Without being bound by theory, it appears that, while healthy individuals and those suffering from ADHD alike do not express antibodies against hDAT, at least that part of the child population suffering from ADHD expresses autoantibodies (aAbs) against one or more epitopes of hDAT after catabolisation. Peptides characterised by SEQ ID NO's 1 - 5 were selected as being most likely to contain hDAT epitopes, and were used in ELISA screens. Children with confirmed ADHD expressed aAbs against each of the peptide fragments characterised by SEQ ID NO's 1 - 5, while children without ADHD did not express detectable levels of such aAbs. The terms 'peptide' and 'peptide fragment' are used interchangeably herein, unless otherwise apparent from the context.

It will be appreciated that it is possible that some patients or subjects suffering from ADHD may not express aAbs against the peptide fragments of the invention, while others may express such fragments, but exhibit few or no symptoms of ADHD, however unlikely either occurrence might be. Thus, the present invention is of assistance to the skilled physician in establishing a diagnosis.

The methods of the present invention may also be targeted according to the genotype of the patient. In particular, the hDAT has a repeat in the polymorphic region, discussed further hereinbelow, wherein the repeat typically occurs either 9 or 10 times. Where both alleles have 10 repeats, the patient is characterised as 10/10. Where the patient has one or two alleles with only 9 repeats, the patient is characterised as 9/x.

In 10/10 patients, higher levels of aAb expression will generally correlate with the need for pharmaceutical treatment. The relevant level is readily established by a skilled physician, but is generally where an ELISA yields a reading above about 1 when measured at 450 nm. The ELISA is preferably as performed and described in Example 1 hereinbelow. Below such a level, non-pharmaceutical treatments may more readily be selected by the skilled physician.

In 9/x patients, the methods of the present invention may be used in the assessment of effectiveness of pharmaceutical treatment, wherein the lower the increase of aAb levels over a selected period, the more successful a treatment is ranked. A suitable period is 6 weeks, but periods of 4 to 12 weeks, and longer, are also useful. Levels of aAbs may not necessarily drop during childhood, but a known ADHD treatment that involves little or no rise in aAbs is also generally associated with reduced symptoms of ADHD over the periods of the invention.

It is preferred that the methods of the present invention be performed on samples obtained from subjects suspected of having ADHD and seeking diagnosis, rather than patients previously diagnosed, although there is no restriction. Patients are preferably under the age of 16, although there is no age restriction.

The present description provides any one of the peptides characterised by SEQ ID NO's 1 - 5, and peptides comprising one or more of said sequences. Peptides comprising only one of any of said sequences are generally preferred. hDAT is a 620 aa polypeptide, and it is preferred that any peptide comprising a sequence of the invention is no more than 100 amino acid residues in length, and preferably somewhere between 50 and 100 residues in length, although peptides of only a few residues in addition those characterised by SEQ ID NO's 1 - 5 are useful, especially where any extra residues are of assistance in binding an ELISA support, or which act as blockers to digestion, for example.

The present invention further provides supports for use with ELISA that have one or more peptides of the invention adsorbed thereto.
the present invention provides strategies for diagnosing and monitoring the efficacy of treatment of ADHD in humans by simple biochemical method based on Enzyme Linked Immuno Sorbent Assay (ELISA). The present invention provides methods an indirect ELISA, for example, for evaluating the level of serum aAb to fragments of hDAT. One possibility is that particular recombinant and/or mutant hDAT are over-expressed in different brain regions of ADHD patients, and that when these allelic variants or subunits are over-expressed the catabolic proteolysis might activate the production of peptide fragments. Should the latter enter the bloodstream, they might be recognised as foreign antigens by the immune system, which would respond by generating detectable amounts of aAb.

The inventors have also discovered that the present invention can monitor the efficacy of "ritalin-like" psycho-stimulants: thereby providing therapeutic strategies which, when combined with the diagnostic methods of the present invention, can be practised more effectively.

In particular, the inventors have discovered that the markers of the present invention can be used to:
(1) verify ADHD diagnosis,
(2) select drug therapies and avoid prescription of wrong medication (for example, "ritalin-like" psycho-stimulant drugs for patients without verified ADHD diagnosis), and/or
(3) monitor the effectiveness and progress of drug therapies.

In one aspect, the present description provides a method of diagnosing hDAT over-expression in a human subject comprising a) detecting in a bodily fluid sample, obtained from the patient, the aAbs recognising at least one hDAT peptide fragment selected from SEQ ID NOs: 1 to 5, wherein statistically significant levels of one or more of said aAbs is indicative of ADHD in said subject.

The terms 'patient' and 'human subject' are used interchangeably herein.

It is preferred that the above method further comprises; b) correlating an excess amount of aAbs to one or more hDAT peptide fragments with ADHD diagnosis; c) optionally correlating an excess amount of aAbs to one or more hDAT peptide fragments with hDAT overexpression in the patient brain; and d) wherein if the said amount of aAb to one or more hDAT peptide fragments exceeds a predetermined value, the patient is diagnosed with ADHD.

It will be appreciated that, unless otherwise specified, or apparent from the context, the sample is obtained from the patient prior to the method of the present invention being carried out, obtaining the sample not being a part of the invention.

The bodily fluid may be any suitable fluid, such as plasma, saliva, tears, urine, and/or spinal fluid.

The term 'statistically significant' has the normal meaning in the art, and is used to indicate that levels of antibody are significant by comparison to an individual not suffering from ADHD considered to be of normal health.

The peptide fragments are preferably adsorbed or otherwise linked to a well or ELISA support, and exposed to the sample from the patient. Antibodies that remain bound to the fragments after eluting and/or washing can then be detected by standard techniques, such as labelled anti-human-Ab antibodies, for example murine anti-human IgG antibodies labelled with horseradish peroxidase. Other suitable antibody detection means are well known to those skilled in the art.

Preferably, the method of the present invention comprises the step of testing body fluids from a said subject for one or more antibodies specific for peptides selected from peptide fragments comprising SEQ ID 2, 4, 5.

In another embodiment, the invention provides a method of diagnosing a patient suffering from ADHD, the method comprising a) identifying a patient suspected of suffering from ADHD; b) directly or indirectly testing a biological fluid from said patient suffering from ADHD for an amount of aAbs to one or more hDAT peptides selected from SEQ ID from 2, 4, 5; c) comparing said amount of aAbs to one or more hDAT peptides with a population norm in apparently healthy human subjects; and d) wherein if the said amount of aAb to one or more hDAT peptides exceeds the population norm, ADHD is diagnosed.

In another embodiment, the method comprises detecting an antibody specific for SEQ ID 2.

In another embodiment, the method comprises detecting an antibody specific for SEQ ID 4.

In another embodiment, the method comprises detecting an antibody specific for SEQ ID 5.

The present invention allows the skilled physician to distinguish between ADHD and ADHD-like syndromes by determining amounts of aAbs, with statistically significant amounts of aAbs being indicative of ADHD.

In a further embodiment, the description provides a method of distinguishing between ADHD and ADHD-like syndromes comprising a) identifying a patient suffering from neuropsychiatric symptoms known to be ADHD-like; b) directly or indirectly testing a biological fluids from said patient for an amount of aAbs to one or more hDAT peptides selected from SEQ ID 1 to 5 or an analogue thereof; c) comparing said amount of aAb to a population norm in apparently healthy human subjects; and d) wherein if the said amount of aAb to one or more hDAT peptides exceeds the population norm, ADHD is diagnosed.

In one embodiment, the method comprises detecting an antibody specific for SEQ ID 1, or an analogue thereof.

In another embodiment, the method comprises detecting an antibody specific for SEQ ID 2, or an analogue thereof.

In another embodiment, the method comprises detecting an antibody specific for SEQ ID 3, or an analogue thereof.

In another embodiment, the method comprises detecting an antibody specific for SEQ ID 4, or an analogue thereof.

In another embodiment, the method comprises detecting an antibody specific for SEQ ID 5, or an analogue thereof.

In another aspect the description provides a kit comprising a) one or more hDAT peptide fragments, or one or more antibodies that specifically bind to hDAT peptide fragments, or one or more nucleic acids that encode hDAT peptide fragments, each bound to a diagnostic substrate or indicator reagent and b) a user's manual for testing a biological fluid for an amount of aAbs to one or more hDAT peptides, wherein said one or more hDAT peptide fragments are selected from: i) SEQ ID1 hDAT peptide fragment or analogue thereof; ii) SEQ ID2 hDAT peptide fragment or analogue thereof; iii) SEQ ID3 hDAT peptide fragment or analogue thereof; iv) SEQ ID4 hDAT peptide fragment or analogue thereof; and v) SEQ ID5 hDAT peptide fragment or analogue thereof.

In another aspect of the invention, there is provided a kit comprising one or more hDAT peptide fragments characterised by a sequence selected from SEQ ID NO's 2, 4, 5, each bound to an ELISA support or substrate. The sequence preferably consists of a sequence selected from SEQ ID NO's 2, 4, 5, this preference also applying, unless otherwise apparent, to other embodiments of the present invention.

There is disclosed herein a method of treating for ADHD in a human subject in need of such treatment comprising a) directly or indirectly testing a body fluid from said subject for a pre-treatment amount of aAbs to one or more hDAT peptide fragment or analogue thereof; b) comparing said pretreatment amount with a population norm; c) if the level of said aAbs to one or more hDAT peptide fragment in excess of said population norm is detected, administering a drug selected from Methylphenidate (Ritalin, Rilatine, Ritalina, Hynidate, MPH, MPD, Methylin, Metadate and Attenta), or/and Atomoxetine (Strattera, Tomoxetin, Attentin) or/and others; and d) if a level of said aAbs to one or more hDAT peptide fragment is in accord of said population norm is detected, avoid administering a drug selected from Methylphenidate (Ritalin, Rilatine, Ritalina, Hynidate, MPH, MPD, Methylin, Metadate and Attenta), or/and Atomoxetine (Strattera, Tomoxetin, Attentin) or/and others. It will be appreciated that, where it is desired to treat a subject diagnosed with ADHD, any medication known in the art may be prescribed by a skilled physician, and that exemplary such medicaments are listed hereinabove.

In an alternative embodiment, the invention provides a method of monitoring the effects of a treatment regime for ADHD in a patient, comprising a) assaying levels of aAb recognising at least one hDAT peptide fragment selected from SEQ ID NOs 2, 4, 5 in a sample obtained from the patient.

Preferably, the method further comprises; b) comparing said pretreatment amount with a population norm; and c) wherein, if the said post-treatment amount of aAb to one or more hDAT peptides exceeds the population norm, the treatment is continued.

In a preferred embodiment, the concentration of naturally-occurring auto-antibodies to DAT is determined using as an antigen a modified synthetic peptide selected from one or more of 2, 4, 5 preferably affinity-adsorbed to immunological ELISA microlevel immuno-plate.

In another aspect of the description, there is provided a method of diagnosing the requirement for pharmaceutical treatment in an ADHD patient having the 10/10 genotype, wherein the method comprises a) identifying a patient suffering from ADHD who has the 10/10genotype; b) detecting levels of aAbs to one or more hDAT peptide fragments selected from SEQ ID NO:1-5 in a sample of body fluid obtained from said patient; c) comparing said level of aAbs to a pre-determined value; d) wherein if the level of aAbs is above the pre-determined value a pharmaceutical treatment is indicated.

In an alternative embodiment, the description provides a method of monitoring the efficacy of pharmacological treatment of an ADHD patient having the 9/x DAT genotype, comprising a) assaying levels of aAb recognising one or more hDAT peptide fragments selected from SEQ ID NO: 1-5 in a sample of biological fluid obtained from the patient, optionally at first and second times, preferably at least separated by 4 weeks, b) comparing the level of aAb with a population norm, and c) continuing the pharmacological treatment if the level of aAb continues to exceed the population norm. In some embodiments, the method also comprises the step of reducing the pharmacological treatment if the level of aAb drops to or approaches the population norm.

While individual peptide fragments selected from SEQ ID NO: 2, 4, 5are useful in the present invention, combinations thereof are also useful, including using peptides fagments selected from SEQ ID 1-5, which is a preferred embodiment, in all aspects.

### BRIEF DESCRIPTION OF THE DRAWINGS

The accompanying drawings illustrate several embodiments of the invention.

The Sequence Listing provides the SEQ IDs of specific hDAT peptides fragments to be used as antigens.

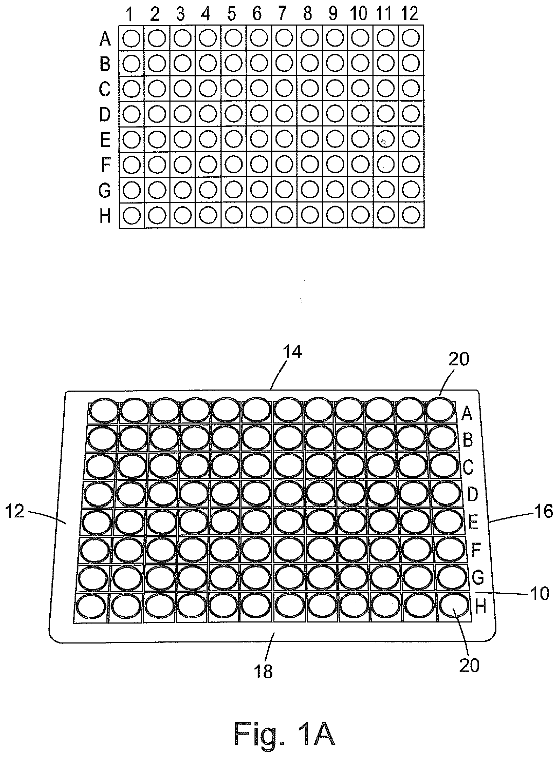

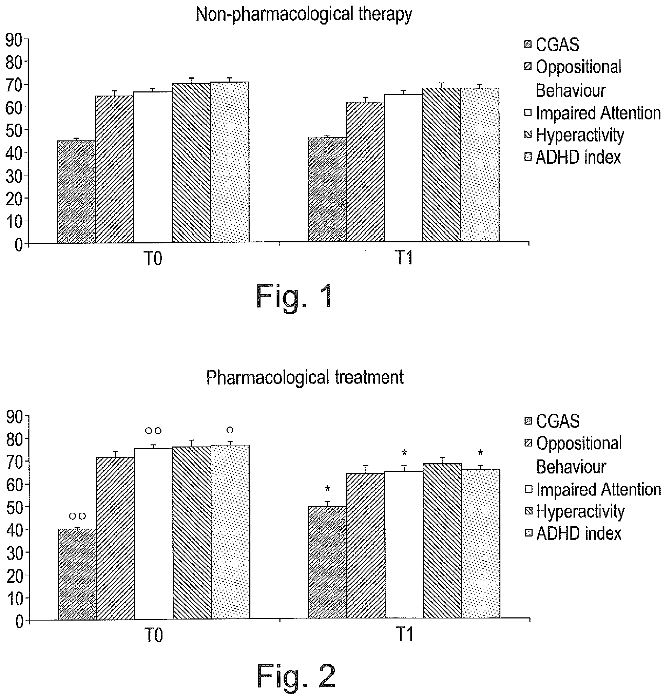

Fig. 1A is a perspective view of a microplate ELISA of the present invention (microlevel plate).
Fig. 1 shows the average psychometric scales in recruited children not assigned to pharmacological therapy (TNF) between time T0 and T1. The symptom scales were filled in by the patients' mothers and the CGAS values were assigned by the patients' clinicians.
Fig. 2 shows the psychometric scales in recruited children assigned to pharmacological therapy (TF) between time T0 and T1. The symptom scales were filled in by the patients' mothers and the CGAS values were assigned by the patients' clinicians.
Fig.3 shows the psychometric scales of 10/10 genotype children (12 subjects) in the TNF group at times T0 and T1. The symptom scales were filled in by the patients' mothers and the CGAS values were assigned by the patients' clinicians.
Fig. 4 shows the psychometric scales of 9/x genotype children (11 subjects) in the TNF group at time T0 and T1. The symptom scales were filled in by the patients' mothers and the CGAS values were assigned by the patients' clinicians.
Fig. 5 shows the psychometric scales of 10/10 genotype children in the TF group at time T0 and T1. The symptom scales were filled in by the patients' mothers and the CGAS values were assigned by the patients' clinicians.
Fig. 6 shows the psychometric scales of 9/x genotype children in the TF group at time T0 and T1. The symptom scales were filled in by the patients' mothers and the CGAS values were assigned by the patients' clinicians.
Fig. 7 shows the levels of aAb directed to hDAT in children not assigned to pharmacological therapy (TNF) at time T0 and T1.
Fig. 8 shows the levels of aAb directed to hDAT in children assigned to pharmacological therapy (TF) at time T0 and T1.,
Fig. 9a shows the levels of aAb directed to hDAT in 10/10 genotype patients in the TNF group, at time T0 and T1.
Fig. 9b shows aAb levels, at T0 and T1, for 9/x genotype patients in the TNF group.
Fig. 10a shows the levels of aAb directed to hDAT in 10/10 genotype patients in the TF group, at time T0 and T1.
Fig. 10b shows the level of aAb directed to hDAT in 9/x genotype patients in the TF group, at time T0 and T1.

As referred to above, the Children's Global assessment Scale (CGAS) is defined by Shaffer D, et al., (1983) A children's global assessment scale (CGAS), Archives of General Psychiatry, 40, 1228-1231.

### DETAILED DESCRIPTION OF THE INVENTION

Referring to Figure 1A, the microlevel plate (10) has a first side (12), a second side (14), a third side (16) and a fourth side (18). Each of the sides (12, 14, 16 and 18) is connected to its adjacent sides to define a rectangular matrix that is made up of 96 wells. The microplate has 96 flat bottom wells, arranged in the standard 8x12 configuration. The walls (12, 14, 16 and 18) are each about 10 mm high, as shown in Fig.1A; the first and third sides (12, 16) are each about 80 mm wide, as shown; the second and fourth sides (14, 18) are each about 122 mm wide as shown.

In further detail, still referring to Fig.1A, these ELISA plates use a solid phase made up of clear polystyrene; high grade for optical transmission. In more detail, the microlevel plate (10) is intended for the semi-quantitative determination of natural antibodies to hDAT in serum and other biological fluids. The concentration is detected using an antigen affinity- adsorbed to the wells' base (20), wherein the antigen is a modified, optionally synthetic, peptide comprising a specific fragment of the DAT sequence.

In more detail, the Sequence Listing provides the sequences of specific hDAT peptide fragments (SEQ ID NOs:1-5) which are useful as antigens in the methods of the present invention. The hDAT peptide fragments may be adsorbed to the wells' base in a standard microlevel immuno-plate or other appropriate matrix. The peptides may be adsorbed, individually or in combination, typically in an amount of between 0.01-10 µg per well. The immuno-plate may then be used for determination of hDAT aAb levels in the blood serum or other suitable biological fluid by standard ELISA.

In a first incubation step, naturally-occurring auto-antibodies (aAb) in the serum sample react with the antigen, i.e. the DAT peptide fragment, bound to the solid phase of the microlevel immuno-plate. After intensive washing, the captured antibodies linked to the antigen (primary immuno-complexes) are detected using monoclonal murine antibodies (mAb) to human IgG conjugated with horseradish peroxidase (HP). The resulting secondary immuno-complex is then semi-quantitatively determined by HRP/TMB-detection reaction. Sulphuric acid stop solution (0.16M) is then added converting the colour from blue to yellow. The intensity of the yellow colour is directly proportional to the concentration of hDAT-directed antibodies in the serum sample. The microplate reader is either a one wavelength (with filters for 450 nm and 405 nm) or a two wavelength (with filters also for 600-650 nm) vertical scan spectrophotometer. A fume cupboard, rocker mixer and microlevel immuno-plate readers are used. The standard operating procedure is all at room temperature and the equipment is substantially similar to conventional ELISA standard manufacture.

Advantages of the present invention include:
1- Reliability
2- Practicability (time, equipment, operator expertise)
3- Correlation between serum levels of the hDAT aAb marker and clinical questionnaires, as shown herein (see below).

Currently, the clinical diagnosis of ADHD is based solely on structured clinical interviews or on questionnaires. Nevertheless, the validity of these criteria for ADHD diagnostics remains a matter for debate. The present invention provides a useful tool for diagnosis, given that we have established that there is a correlation between naturally-occurring auto-antibodies to specific peptide fragments of human dopamine transporter (hDAT-aAb), for example in serum, with ADHD. Results are available quickly, allowing rapid diagnosis of ADHD in a clinical setting, thereby allowing the commencement of treatment of patients in less time than that required for completion of questionnaires.

In particular, it has been found that alleles of the DAT, also referred to as DAT1 and SLC6A3, gene comprising 9 repeats (9/x genotype) or 10 repeats (10/10 genotype) in the polymorphic region UTR region of the promoter are associated with the over-expression of recombinant and/or mutant hDAT in ADHD patients. The term "9/x genotype" refers to a genotype comprising at least one 9-repeat allele of the DAT gene, while the term "10/10 genotype" refers to a carrier of two 10-repeat alleles of SLC6A3. The genotype of ADHD patients may be determined using a buccal swab and PCR analysis of the cells collected. This is illustrated in the accompanying Comparative Example.

It has been found that specific peptide fragments may be used as antigen sequences to detect hDAT-aAb in samples of biological fluids obtained from subjects with ADHD. These peptide fragments (SEQ ID NO:1-5) may be used to detect hDAT-aAbs in patients with either the 9/x DAT genotype or the 10/10 DAT genotype.

More specifically, it has been found that raised levels of aAbs are diagnostic of ADHD in 10/10 DAT genotype patients suspected of suffering from ADHD.

This disclosure also includes:
A) A method of diagnosing hDAT overexpression in a human subject comprising:
   a) detecting in a sample of body fluid obtained from the human subject, directly or indirectly, the amount of naturally-occurring auto-antibodies (aAbs) to one or more hDAT peptide fragments selected from SEQ ID NOs:1 to 5, or analogues thereof;
   b) correlating the amount of aAbs to one or more hDAT peptide fragments with an ADHD diagnosis; and
   c) optionally correlating an excess amount of aAbs to one or more hDAT peptide fragments with hDAT overexpression in the patient brain;
   wherein if the said amount of aAb to one or more hDAT peptide fragments exceeds a predetermined value, the patient is diagnosed with ADHD.
B) A method of diagnosing ADHD in a patient, the method comprising:
   a) identifying a patient suspected of suffering from ADHD;
   b) directly or indirectly testing a biological fluid obtained from said patient for an amount of aAbs to one or more hDAT peptide fragments selected from SEQ ID from 1 to 5 or an analogue thereof; and
   c) comparing said amount of aAbs to one or more hDAT peptide fragments with a population norm in apparently healthy human subjects;
   wherein if the amount of aAb to one or more hDAT peptide fragments exceeds the population norm, ADHD is diagnosed.
C) The method according of A or B, comprising detecting an antibody specific for SEQ ID 1, or an analogue thereof, and/or detecting an antibody specific for SEQ ID 2, or an analogue thereof, and/or detecting an antibody specific for SEQ ID 3, or an analogue thereof, and/or an antibody specific for SEQ ID 4, or an analogue thereof, and/or detecting an antibody specific for SEQ ID 5, or an analogue thereof.
D) A method of distinguishing between ADHD and ADHD-like syndromes comprising:
   a) identifying a patient suffering from neuropsychiatric symptoms known to be ADHD-like;
   b) directly or indirectly testing a biological fluids from said patient for an amount of aAbs to one or more hDAT peptide fragments selected from SEQ ID NOs:1 to 5, or an analogue thereof; and
   c) comparing said amount of aAb to one or more hDAT peptide fragments with a population norm in apparently healthy human subjects;
   wherein if the said amount of aAb to one or more hDAT peptide fragments exceeds the population norm, ADHD is diagnosed.
E) The method of D, comprising detecting an antibody specific for SEQ ID 1, or an analogue thereof, and/or detecting an antibody specific for SEQ ID 2, or an analogue thereof, and/or detecting an antibody specific for SEQ ID 3, or an analogue thereof, and/or detecting an antibody specific for SEQ ID 4, or an analogue thereof, and/or detecting an antibody specific for SEQ ID 5, or an analogue thereof.
F) A kit comprising:
   a) one or more hDAT peptide fragments, or one or more antibodies that specifically bind to hDAT peptide fragments, or one or more nucleic acids that encode hDAT peptide fragments, each bound to a diagnostic substrate or indicator reagent; and
   b) a user's manual for testing a biological fluid for an amount of nAbs to one or more hDAT peptides,
   wherein said one or more hDAT peptide fragments are selected from:
   i) SEQ ID1 hDAT peptide fragment or analogue thereof;
   ii) SEQ ID2 hDAT peptide fragment or analogue thereof;
   iii) SEQ ID3 hDAT peptide fragment or analogue thereof;
   iv) SEQ ID4 hDAT peptide fragment or analogue thereof; and
   v) SEQ ID5 hDAT peptide fragment or analogue thereof.
G) A method of monitoring the treatment of ADHD in a human subject comprising:
   a) directly or indirectly testing a sample of body fluid from said subject for a post-treatment amount of aAb to one or more hDAT peptide fragments or analogue thereof;
   b) comparing said post-treatment amount with a population norm;
   c) wherein, if the said post-treatment amount of aAb to one or more hDAT peptide fragments exceeds the population norm, the treatment is continued.

The following Examples are illustrative of the present invention, and are not limiting thereon.

### EXAMPLES

### Comparative Example

### Determination of patient DAT genotype

The genotype of subjects was determined by taking a buccal swab and analysing the throat cells collected using standard PCR. In particular, Genomic DNA was prepared from buccal swab samples by using the BuccalAmp™ DNA Extraction Kit, following the manufacturer instructions (Epicentre, USA). Briefly, after collecting buccal cells, the swab end was placed into a tube containing QuickExtract DNA extraction solution and rotated a minimum of five times. The tube was vortex mixed for 10 seconds and incubated at 65°C for 1 minute. After vortex mix for 15 seconds, the tube was transferred to 98°C and incubated for 2 minutes. After vortex mix for 15 seconds, the DNA was stored at -20°C. The yield of DNA is usually between 2-14 ng/µl.

The 3'-UTR repeated sequence of the DAT1 gene was amplified by the polymerase chain reaction (PCR). The primer sequences employed were 5'-TGT GGT GTA GGG AAC GGC CTG AG-3' (DAT1-F; SEQ ID NO:6) and 5'-CTT CCT GGA GGT CAC GGC TCA AGG-3' (DAT1-R; SEQ ID NO:7). The PCR amplification was carried out in a final volume of 50µl containing 3µl of genomic DNA prepared using the Buccal Amp DNA extraction kit, 1.5mM MgCl2, 200µM dNTP, 50mM KCI, 10mM Tris-HCI (pH8.3), 0.25µM of each primer, and 1 U of Promega Taq DNA polymerase. The PCR amplification was performed for 35 cycles consisting of 94°C for 45 s, 57°C for 30 s, and 72°C for 30 s. The genotype was estimated from the size of the PCR product analyzed by electrophoresis on 6% acrylamide gels stained with ethidium bromide.

### Example 1

### Combined use of hDAT-aAb Test and questionnaires

This experiment was to verify any correlation between hDAT-aAb levels in the serum, as an innovative diagnostic ADHD marker, and the scores obtained in clinical questionnaires. The study set out to measure, by ELISA, the blood levels of hDAT-directed naturally-occurring auto-antibodies (hDAT-aAb levels) that targeted specific epitopes of hDAT, and to correlate these levels with scores of ADHD symptoms.

This study included 61 children with ADHD, of which 1/3 were assigned to a pharmacological treatment and 2/3 were assigned to a non-pharmacological therapy. All subjects met DSM-IV criteria for ADHD and IQ over 85. Two blood samples were withdrawn: one at recruitment (T0 basal, early in the morning); another after a 6-week period (T1, late in the morning). In the first step, we evaluated the hDAT-aAbs levels, obtained by measuring optical density at wavelength 450nm (OD₄₅₀) in ELISA assays, in the ADHD and control children. In the second step, we ran a correlation analysis between hDAT-aAbs levels at T0 and T1 in ADHD children, using results from the above-described ELISA assay and using all 5 peptide fragments having SEQ ID NOs: 1 - 5, and scores obtained in questionnaires run by parents and by teachers of the recruited children. In the third step, we characterised the DAT genotype of the patient as to the 9-repeat or 10-repeat polymorphic alleles and considered the results based on this information.

In brief, the ELISA assay was performed by adsorbing modified synthetic fragments of DAT (DAT-Asp) to immunological ELISA microplates by affinity. In a first incubation step, natural antibodies in the sample were reacted with the solid phase bound DAT-Asp peptide. After intensive washing the captured antibodies are detected using monoclonal murine antibodies (MCMAB) to human IgG conjugated with horseradish peroxidase (HP). The formed immunocomplex is then quantitatively determined followed by intensive washing in a third incubation step via the HRP/TMB-detection reaction. An acidic stopping solution is then added. The colour converts from blue to yellow. The intensity of the yellow color is directly proportional to the concentration of DAT antibodies in the sample.

In more detail, a working buffer solution was prepared by diluting 20ml of 20-fold concentrate of phosphate-saline buffer with Tween-20 (20XWASHBUF) in distilled water to reach a volume of 400ml. Serum samples were diluted by mixing 20 µl of serum with 80 µl of serum dilution buffer (SDB; Bovine serum albumin (Cat. Nº) # A7511, Sigma, USA) - 0.5 g, 20-fold concentrate of phosphate-buffered saline solution - 5 ml; Ponceau C stain (Cat. number # P3504, Sigma, USA) - 0.05 g; Thimerosal (Cat. Nº # T8784, Sigma, USA) - 0.02 g, Sodium azide (Cat. Nº) S8032, Sigma, USA) - 0.05 g; distilled water, 1000 ml). The conjugate working solution (Mouse Anti-Human IgG Monoclonal antibody, Hrp Conjugated (Cat. Nº # A8667, Sigma, USA), liquid) was prepared immediately before use by mixing 1 ml of "conjugate" with 0.01 ml "Anti-Mouse IgG HRP x100" for each strip of the microplate, wherein "conjugate" is the solution for dilution of conjugate and "Anti-Mouse IgG HRP x100" is 100x concentrate of anti-mouse IgG HRP conjugate. The substrate working solution was prepared immediately before use by mixing 0.5 ml of citrate-phosphate buffer (CPB; - disodium hydrogen phosphate (Cat. Nº # S9763, Sigma, USA) - 26.9 g, citric acid (Cat. Nº # 251275, Aldrish, USA) - 4.51 g, distilled water, 1000 ml) with 0.5 ml of 3,3',5,5'-tetramethyl benzidine (TMB; 3, 3', 5, 5'-tetramethylbenzidine - chromogen - 3, 3', 5, 5' - tetramethylbenzidine (Cat. Nº # 09743, Fluka, USA), liquid).

The following reagents were also used:
- a positive control sample (K+): liquid human serum at 1:1000 dilution, inactivated by heating in the water bath at the temperature ranging from 54 to 56 C° for 3h, containing class G antibodies against DAT antigen and Ponceau G stain (Cat. Nº # P3504, Sigma, USA) containing no HBsAg, antibodies to HIV-1, HIV-2, hepatitis C, Treponema pallidum. The serum was tested for class G antibodies to DAT antigen by western blot using purified native antigen of DAT human protein;
- a negative control sample (K-): liquid human serum at 1:1000 dilution, inactivated by heating in the water bath at the temperature ranging from 54 to 56 C° for 3h, containing class G antibodies against DAT antigen and containing bromothymol blue stain (Cat. Nº # B7271, Sigma, USA), containing no HBsAg, antibodies to HIV-1, HIV-2, to hepatitis C, *Treponema pallidum.* The serum was tested for absence of class G antibody to DAT antigen by western blot using purified native antigen of DAT human protein;
- serum incubation buffer (SIB) - bovine serum albumin (Cat. Nº # A7511, Sigma, USA) - 0.5 g; 20-fold concentrate of phosphate-buffered saline solution - 5 ml, bromophenol blue (Cat. Nº # B0126, Sigma, USA) - 0.05 g; Thimerosal (Cat. Nº # T8784, Sigma, USA) - 0,02 g, sodium azide (Cat. Nº S8032, Sigma, USA) - 0.05 g, distilled water, 1000 ml;
- PBS-T: 20-fold concentrate of phosphate-buffered saline solution - sodium chloride (Cat. Nº # 204439, Aldrish, USA) -160 g, disodium hydrogen phosphate (Cat. Nº # S9763, Sigma, USA) - 43.4 g, potassium phosphate monobasic (Cat. Nº # 0662, Sigma, USA) - 4.0 g, potassium chloride (Cat. Nº # 449989, Aldrish, USA) - 4.0 g; thimerosal (Cat. Nº # T8784, Sigma, USA) - 0.05 g, distilled water, 1000 ml.

Two blank control wells, containing 100 µl of SIB each, two positive control sample wells (100 µl each) and two negative control sample wells (100 µl each) were prepared. Test wells were prepared by filling each with 90 µl of SIB and 10 µl of previously diluted serum samples, so that the final working dilution of the serum samples was 1:50. The microplate was sealed with protective film and incubated in a rocker at 37±1 °C for 30 minutes at 500 RPM or in thermostat at 37±1 °C for one hour. After incubation, the microplates were rinsed five times with working solution by:
- Elutriating the content of wells into a container with a disinfecting solution;
- Filling each well with 300 µl of washing buffer solution and mixing in a shaker for 5 seconds;
- Elutriating the content of the wells into a container with a disinfecting solution;
- During each elutriation, thoroughly removing residual liquid from the well by slightly rapping the upturned microplate against filter paper.

After rinsing and wiping, each well was filled with 100 µl of conjugate working solution. The microplate was sealed with protective film and incubated on a rocker at 37±1 °C for 30 minutes at 500 RPM or in thermostat at 37±1 °C for 30 minutes. The microplates were then rinsed five times with working solution and the moisture removed, as detailed above. 100 µl of substrate solution was inserted into each well, and the microplate incubated on a rocker at 37±1 °C for 10 minutes at 500 RPM or in thermostat at 37±1 °C for 10 minutes. The reaction was stopped by adding 100 µl of 2N solution of hydrochloric acid and mixing thoroughly.

The optical density of the contents of the wells of the microplate was calculated using a spectrophotometer at wavelength 450 nm within 15 minutes of stopping the reaction.

The results demonstrated that there were measurable levels of hDAT-aAbs in ADHD children, but that levels were almost undetectable in control children. The basal levels at T0 were higher in patients afterwards assigned to undergoing Methylphenidate (MPH) treatment, compared to basal levels at T0 in those patients afterwards assigned to undergoing behavioural therapy, or to those afterwards assigned to undergoing no therapy. Specifically, basal levels at T0 were higher in patients carrying the DAT 10/10 genotype and in need of pharmacological treatment.

Levels at T1 were higher than those at T0 in patients afterwards assigned to undergoing behavioural therapy or to undergoing no therapy, suggesting that a "physiological" rise occurs between early and late morning. After a 6-week period of methylphenidate treatment, the levels at T1 did not differ when compared to a similar period of behavioural (or no) therapy. Specifically, a "physiological" rise in levels at T1 was abolished in patients carrying the 9/x genotype, who were observed to experience the most pronounced beneficial effects of pharmacological treatment.

The hDAT-aAbs levels in serum of patients under behavioural therapy correlated with the questionnaires compiled by parents and by teacher (r > 0.34), but only for children with a DAT 10/10 genotype (r > 0.53).

These data confirm (1) that ADHD children with a DAT 10/10 genotype have abnormal serum levels of hDAT-aAbs, which correlate with severity of symptoms, and (2) that the most pronounced beneficial effects of pharmacological treatment are linked to and/or can be monitored by the lack of a "physiological" rise in levels at T1 in patients carrying a DAT 9/x genotype. This demonstrates that determination of serum hDAT-aAbs is effective to confirm diagnosis of ADHD and/or to monitor the efficacy of MPH treatment.

### 1) Symptom scales: CGAS and Conners'

The average values of symptom scales filled in by the patients' mothers and of CGAS values assigned by the clinician show, between T0 and T1, a globally stable profile of ADHD symptoms in the group of children under non-pharmacological therapy (32 subjects), as represented in Fig.1. The Student t-test analysis did not demonstrate any significant results.

In the group of children placed under pharmacological treatment (14 subjects), the average values of symptom scales filled in by their mothers and of CGAS values assigned by the clinician showed more severe ADHD symptoms, compared to those subjects who did not need pharmacological therapy. A period of 6 weeks under MPH treatment led to an important reduction of ADHD symptoms between T0 and T1; this reduction of symptoms was significant for CGAS (t (26)=3.67), for the attention deficit sub-scale (t (26)=3.13), and for the ADHD index (t (26)=3.27), as the Student t-test analysis demonstrated (Fig. 2).

The Student t-test analysis that compared Conners' scales and CGAS values between the two groups (TNF vs TF subjects) revealed:
- T0: a significant difference in score for CGAS values (t(44)=3.79), for the attention deficit sub-scale (t(44)=2.92) and for the ADHD index (t(44)=2.10). This finding suggested that subjects assigned to the MPH treatment group showed indeed more severe ADHD symptoms compared to subjects who did not need pharmacological therapy.
- T1: there were no significant differences. This indicates that, after 6 weeks of pharmacological treatment, these subjects showed an important reduction in symptoms, so that they could no longer be distinguished from subjects who did not take any pharmacological therapy.

### Role of genotype

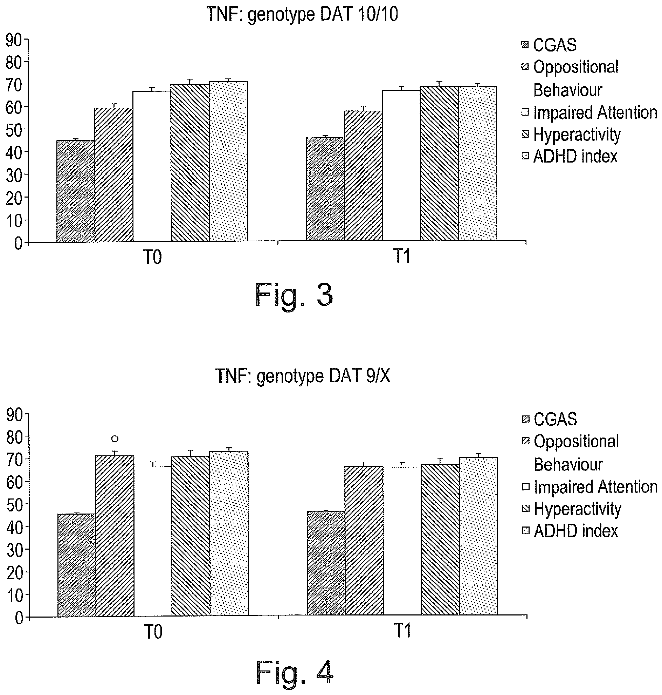

By splitting the subjects who did not take pharmacological therapy into two subgroups, namely carriers of a DAT 10/10 (12 subjects) vs 9/x (11 subjects) genotype, we observed that:
- genotype 10/10: ADHD symptoms were unchanged from T0 to T1 (Fig. 3), suggesting that this genetic component may cause the expression of a phenotype that does not reveal any spontaneous improvement.
- genotype 9/x: according to their mothers and in relation to oppositional behaviour, these subjects showed a more severe symptomatology at T0, when compared to those carrying a 10/10 genotype (t(21)=2.39). At T1, these subjects showed a slight reduction of all symptoms; however, the oppositional behaviour was still more severe compared to subjects carrying a 10/10 genotype (Fig. 4).

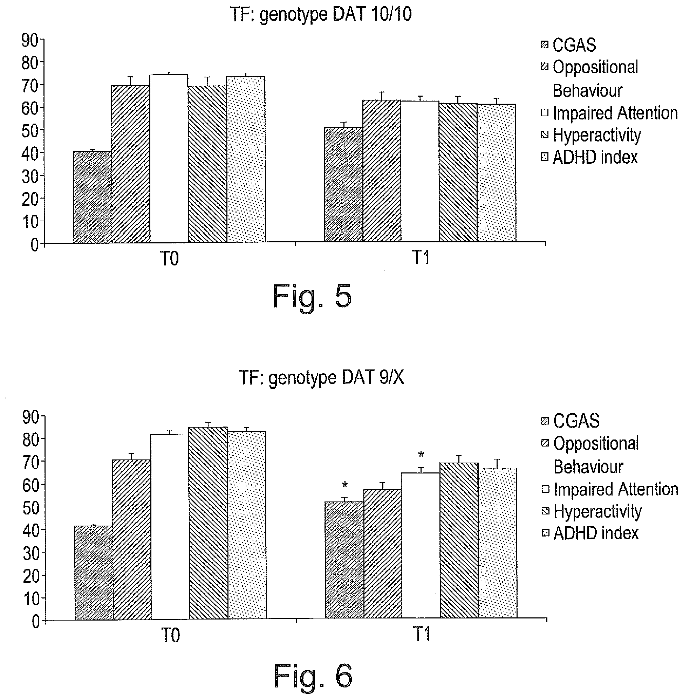

By splitting the subjects who needed to take pharmacological treatment into two subgroups, namely in carriers of a DAT 10/10 (3 subjects: Fig. 5) vs 9/x (4 subjects: Fig. 6) genotype, we observed that:
- genotype 10/10: subjects with these alleles showed a reduction of ADHD symptoms according to CGAS and Conners' scales values between T0 and T1; this reduction was however slight and not statistically significant.
- genotype 9/x: subjects with at least one DAT9 allele showed symptoms of inattention, hyperactivity and ADHD Index which were slightly more severe than those found in subjects with a 10/10 genotype, at T0. They, however, showed a better response to MPH; in fact, we observed a significant reduction of CGAS (t(6)=2.80) and attention deficit sub-scale (t(6)=2.97) as the Student t-test analysis demonstrated.

### 2) Antibody level

All ADHD children revealed measurable levels of DAT-directed autoantibodies (hDAT-aAbs), whereas we could observe no appreciable levels of these antibodies in healthy children.

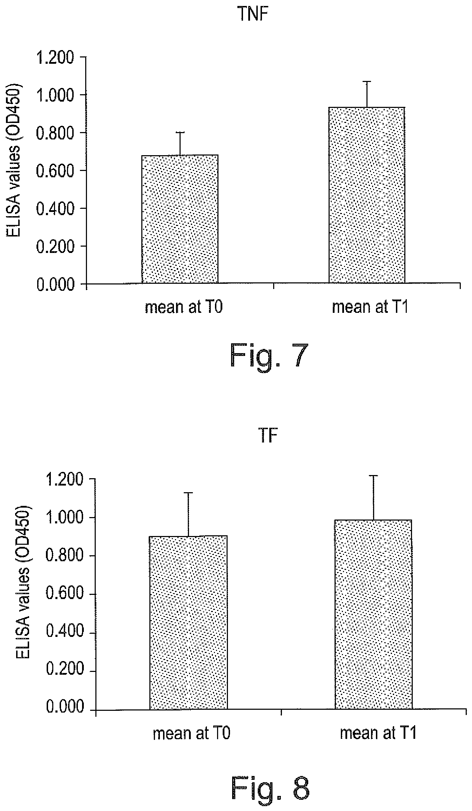

The average aAbs levels in subjects who did not take pharmacological therapy showed a non-significantly increased value from T0 to T1 (Fig. 7). This unexpected rise could represent either a methodological error, or rather a "physiological" phenomenon. In both cases, this might be due to the fact that the two blood samples were not withdrawn in the same conditions; at T0 the sample was taken early in the morning, and could be therefore representative of the basal antibody level; conversely, the sample withdrawn at T1 could be used to estimate the "physiological" levels reached in the late morning (and/or after having a breakfast).

The average aAbs level in subjects under pharmacological treatment showed, between T0 and T1 time, a quite stable value (Fig 8). In these subjects, after 6 weeks of MPH treatment, we could not find an increasing level, as we could have expected based on the findings for the non-pharmacological therapy group. Noteworthy, at T1 time, patients were not taking the morning dose of Ritalin, therefore any differences between pharmacological therapy (TF) and non-pharmacological therapy (TNF) subjects were indicative of the continued usage effects of the drug itself.

When comparing the two groups (TF vs TNF), we can observe that subjects then assigned under pharmacological treatment presented, at T0, higher antibody levels compared to the group then assigned under non pharmacological therapy. These data allow us to suppose higher basal antibody levels in subjects suffering from a more severe type of ADHD, who are actually in the need of a pharmacological approach. On the other hand, at T1, this difference was no longer evident because aAbs levels were similar in the two groups. Noteworthy, also behavioural profiles had become similar between the two groups (Fig.1 and 2). This information can lead us to suppose a close relationship between behaviour and the immune marker: subjects may have similar aAbs levels when also showing a similar behavioural profile (after 6 weeks) while aAbs levels did differ when the symptoms also differed (T0). Thus, levels could be used to monitor the efficacy of MPH.

### Role of genotype

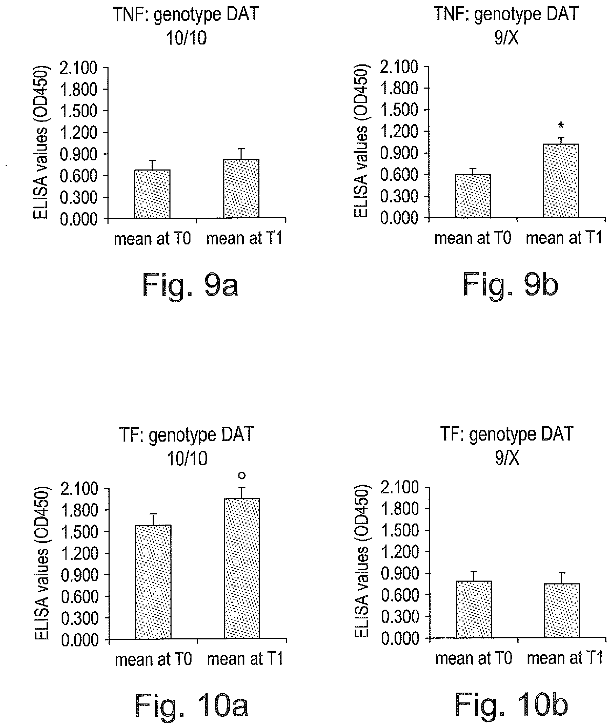

By splitting the subjects who did not take pharmacological therapy into the two different DAT genotypes, we observed that:
- T0: basal aAbs levels were similar for subjects carrying a 10/10 genotype (Fig. 9a) when compared to 9/X subjects (Fig. 9b);
- T1: subjects carrying a 9/x genotype showed a significant increase of antibody levels from T0 to T1 (t(20)=2.01). Moreover, at T1, the aAbs level in subjects carrying a 9/X genotype did not significantly differ compared to the level in patients with a 10/10 genotype (t(21)=0.67). This could suggest that the slight spontaneous improvement, observed in the oppositional behaviour of subjects with a 9/x genotype, could be related to such a rise of levels detected at T1 compared to T0.

By splitting the subjects under MPH treatment into the two different DAT genotypes, we observed that:
- at T0: the basal autoantibody level of DAT 10/10 subjects (Fig. 10a) was almost double when compared to the basal aAbs level of 9/x subjects (Fig 10b). This suggests that subjects with a genotype "at risk" could be also characterised by a very high basal antibody level.
- at T1: for the 10/10 genotype, the aAbs level was significantly increased when compared to that of 9/x subjects (t(5)=2.59). For both the two possible DAT genotypes, there were no significant differences when the T1 level was compared to the T0 level. Besides, the difference in the levels between the two genotypes was still almost double; this confirms that a high level, both basal and after treatment, may occur in DAT 10/10 carriers, i.e. those with a genetic risk for ADHD and that may also show a lower drug response (Fig. 5).Conversely, the level is surprisingly not followed by an increase at T1 in the group under MPH treatment, independently from genotype.

Considering the comparison between the two therapeutic groups, we observed that:
1) Among subjects carrying a DAT 10/10 genotype, those placed under pharmacological treatment showed a doubled level when compared to those that were not placed under a pharmacological therapy. This was the case, both at T0 (basal level) and significantly at T1 (t(13)=2.06) after 6 weeks of therapy;
2) subjects with a DAT 9/x genotype did not show any difference in basal level (T0) between the two therapeutic groups. In the sample withdrawn at T1, their levels revealed a significant "physiological" increase but only in the group of TNF subjects (Fig. 9b) and interestingly no such increase due to a 6-week MPH treatment.

### 3) Correlations between behavioural scales and aAbs levels

Antibody level at T0 or T1 and the behavioural scales filled in by the parents at T0 or T1 showed a significant relationship. A significant correlation was found between levels, both at T1 and basal, and the values of inattention suggested by fathers. In addition, a significant trend was found between basal levels and hyperactivity values suggested by teachers. These correlations were found in subjects under non-pharmacological therapy (Table 1). This means that, when the inattention level suggested by the fathers and/or the motor behaviour suggested by the teachers do increase, the autoantibodies against DAT circulating in the blood also increase.

By splitting into the two genotypes, the correlation concerning the father's opinion about the attention disorder disappears for the 9/x subgroup. On the other hand, for the 10/10 subgroup, the correlations were confirmed and extended. A statistically significant correlation emerged between levels (both basal and at T1) and all the behavioural scales suggested by fathers. Moreover, we found a significant relationship between levels (both basal and at T1) and the oppositional behaviour suggested by teachers (Tables 2 and 3).

Correlations between autoantibodies and behavioural scales for the pharmacological treatment group did not show any statistically significant correlation (Table 4).

**Table 1.**

| | TNF | | | | | | | | | | | | |
|---|---|---|---|---|---|---|---|---|---|---|---|---|---|
| | CGAS | Conners' Mother | | | | Conners' Father | | | | Conners' Teacher | | | |
| | | oppositional behaviour | impaired attention | hyperactivity | ADHD index | oppositional behaviour | impaired attention | hyperactivity | ADHD index | oppositional behaviour | impaired attention | hyperactivity | ADHD index |
| mean T0 | 0.091 | -0.056 | 0.158 | -0.122 | -0.007 | 0.106 | 0.514 | 0.133 | 0.269 | 0.151 | 0.253 | 0.329 | 0.295 |
| mean T1 | 0.057 | 0.102 | 0.047 | 0.013 | 0.048 | 0.078 | 0.010 | -0.042 | 0.021 | | | | |
| c T0 vs Ab T1 | 0.032 | 0.005 | 0.123 | -0.004 | 0.016 | 0.176 | 0.419 | 0.202 | 0.274 | 0.093 | 0.123 | 0.246 | 0.185 |
| 0.338 | | | | | | | | | | | | | |

**Table 2**

| | TNF 10/10 | | | | | | | | | | | | |
|---|---|---|---|---|---|---|---|---|---|---|---|---|---|
| | CGAS | Conners' Mother | | | | Conners' Father | | | | Conners' Teacher | | | |
| | | oppositional behaviour | impaired attention | hyperactivity | ADHD index | oppositional behaviour | impaired attention | hyperactivity | ADHD index | oppositional behaviour | impaired attention | hyperactivity | ADHD index |
| mean T0 | -0.349 | 0.237 | 0.374 | -0.150 | -0.212 | 0.610 | 0.740 | 0.545 | 0.543 | 0.578 | 0.302 | 0.400 | 0.399 |
| mean T1 | -0.002 | 0.184 | -0.157 | -0.243 | -0.338 | 0.193 | -0.157 | -0.243 | -0.355 | | | | |
| c T0 vs Ab T1 | -0.263 | 0.112 | 0.103 | -0.131 | -0.404 | 0.434 | 0.543 | 0.457 | 0.325 | 0.503 | 0.324 | 0.448 | 0.419 |
| 0.530 | | | | | | | | | | | | | |

**Table 3**

| | TNF 9/X | | | | | | | | | | | | |
|---|---|---|---|---|---|---|---|---|---|---|---|---|---|
| | CGAS | Conners' mother | | | | Conners' father | | | | Conners' Teacher | | | |
| | | oppositional behaviour | impaired attention | hyperactivity | ADHD index | oppositional behaviour | impaired attention | hyperactivity | ADHD index | oppositional behaviour | impaired attention | hyperactivity | ADHD index |
| mean T0 | 0.179 | -0.300 | 0.233 | -0.261 | 0.090 | -0.428 | 0.267 | -0.117 | 0.020 | -0.154 | 0.895 | 0.070 | 0.351 |
| mean T1 | -0.225 | 0.103 | 0.315 | 0.261 | 0.286 | -0.129 | 0.120 | 0.033 | 0.082 | | | | |
| c T0 vs Ab T1 | -0.155 | -0.154 | 0.230 | 0.173 | 0.188 | -0.084 | 0.232 | 0.171 | 0.233 | -0.533 | 0.490 | -0.270 | -0.097 |
| 0.550 | | | | | | | | | | | | | |

**Table 4**

| | TF | | | | | | | | | | | | |
|---|---|---|---|---|---|---|---|---|---|---|---|---|---|
| | CGAS | Conners' Mother | | | | Conners' Father | | | | Conners' Teacher | | | |
| | | oppositional behaviour | impaired attention | hyperactivity | ADHD index | oppositional behaviour | impaired attention | hyperactivity | ADHD index | oppositional behaviour | impaired attention | hyperactivity | ADHD index |
| mean T0 | 0.372 | -0.306 | -0.114 | -0.108 | - 0.386 | -0.272 | -0.234 | 0.083 | - 0.230 | 0.187 | 0.130 | 0.194 | - 0.002 |
| mean T1 | 0.142 | 0.248 | 0.098 | 0.182 | 0.089 | 0.283 | 0.084 | 0.200 | 0.114 | | | | |
| 0.497 | | | | | | | | | | | | | |

## Claims

1. A method for assisting in the diagnosis of attention deficit hyperactivity disorder (ADHD) in a subject, comprising assaying a sample of a biological fluid obtained from the subject for the presence of antibodies capable of recognising at least one peptide selected from those **characterised by** SEQ ID NO's 2, 4, 5.

2. A method for identifying a subject suspected of having ADHD and/or confirming a diagnosis of ADHD, comprising determining whether a sample of biological fluid obtained from said subject contains a statistically significant level of antibodies capable of recognising at least one peptide selected from those **characterised by** SEQ ID NO's 2, 4, 5.

3. A method according to claims 1 or 2, wherein the subject has 10 repeats in the polymorphic region of both alleles for the human dopamine transporter (hDAT).

4. A method according to any of claims 1 to 3 wherein the peptide recognised is **characterised by** SEQ ID NO 2.

5. A method according to any of claims 1 to 3 wherein the peptide recognised is **characterised by** SEQ ID NO 4.

6. A method according to any of claims 1 to 3 wherein the peptide recognised is **characterised by** SEQ ID NO 5.

7. A method for assessing the effectiveness of a treatment regime for ADHD in a subject, comprising assaying a sample of a biological fluid obtained from the subject for the presence of antibodies capable of recognising at least one peptide selected from those **characterised by** SEQ ID NO's 2, 4, 5.

8. A method according to claim 7, wherein a second assessment is performed at least four weeks after the first assessment.

9. A method according to claim 7 or 8, wherein the subject has 9 repeats in the polymorphic region of one or both alleles for the human dopamine transporter (hDAT).

10. A peptide which is selected from SEQ ID NO's 2, 4, 5.

11. A support for use with ELISA that has one or more peptides selected from SEQ ID NO's 2, 4, 5 adsorbed thereto.

12. A kit comprising one or more peptides **characterised by** a sequence selected from SEQ ID NO's 2, 4, 5, each bound to an ELISA support or substrate.

## Patentansprüche

1. Verfahren zum Unterstützen bei der Diagnose von Aufmerksamkeitsdefizit-/Hyperaktivitätsstörung (ADHS) bei einer Testperson, das umfasst: Prüfen einer Probe einer biologischen Flüssigkeit von der Testperson auf das Vorliegen von Antikörpern, die in der Lage sind, mindestens eines von den Peptiden zu erkennen, die durch SEQ ID Nrn. 2, 4, 5 gekennzeichnet sind.

2. Verfahren zum Identifizieren einer Testperson mit Verdacht auf ADHS und/oder zum Bestätigen einer ADHS-Diagnose, das umfasst: Bestimmen, ob eine Probe einer biologischen Flüssigkeit, die von der Testperson erhalten wird, einen statistisch signifikanten Pegel von Antikörpern enthält, die in der Lage sind, mindestens eines von den Peptiden zu erkennen, die durch SEQ ID Nrn. 2, 4, 5 gekennzeichnet sind.

3. Verfahren gemäß den Ansprüchen 1 oder 2, wobei die Testperson über 10 Wiederholungen in der polymorphen Region beider Allele für den humanen Dopamintransporter (hDAT) verfügt.

4. Verfahren gemäß einem der Ansprüche 1 bis 3, wobei das erkannte Peptid durch SEQ ID Nr. 2 gekennzeichnet ist.

5. Verfahren gemäß einem der Ansprüche 1 bis 3, wobei das erkannte Peptid durch SEQ ID Nr. 4 gekennzeichnet ist.

6. Verfahren gemäß einem der Ansprüche 1 bis 3, wobei das erkannte Peptid durch SEQ ID Nr. 5 gekennzeichnet ist.

7. Verfahren zum Abschätzen der Wirksamkeit einer Behandlungsmethode gegen ADHS bei einer Testperson, das umfasst: Prüfen einer Probe einer biologischen Flüssigkeit, die von der Testperson erhalten wird, auf das Vorliegen von Antikörpern, die in der Lage sind, mindestens eines von den Peptiden zu erkennen, die durch SEQ ID Nrn. 2, 4, 5 gekennzeichnet sind.

8. Verfahren gemäß Anspruch 7, wobei eine zweite Abschätzung mindestens vier Wochen nach der ersten Abschätzung ausgeführt wird.

9. Verfahren gemäß Anspruch 7 oder 8, wobei die Testperson über 9 Wiederholungen in der polymorphen Region eines oder beider Allele für den humanen Dopamintransporter (hDAT) verfügt.

10. Peptid, das aus SEQ ID Nrn. 2, 4, 5 ausgewählt wird.

11. Träger zur Verwendung mit ELISA mit einem oder mehreren daran adsorbierten Peptiden, die aus SEQ ID Nrn. 2, 4, 5 ausgewählt werden.

12. Kit, das ein oder mehrere Peptide umfasst, die durch eine Sequenz gekennzeichnet sind, die aus SEQ ID Nrn. 2, 4, 5 ausgewählt werden, wobei jedes an einen ELISA-Träger oder ein -Substrat gebunden ist.

## Revendications

1. Procédé pour aider dans le diagnostic du trouble d'hyperactivité avec déficit de l'attention (THADA) chez un sujet, comprenant l'analyse d'un échantillon de liquide biologique obtenu du sujet pour la présence d'anticorps capables de reconnaître au moins un peptide choisi parmi ceux **caractérisés par** SEQ ID NO : 2, 4, 5.

2. Procédé pour identifier un sujet suspecté d'être atteint de THADA et/ou pour confirmer un diagnostic de THADA, comprenant la détermination si un échantillon de liquide biologique obtenu dudit sujet contient un taux statistiquement significatif d'anticorps capables de reconnaître au moins un peptide choisi parmi ceux **caractérisés par** SEQ ID NO : 2, 4, 5.

3. Procédé selon les revendications 1 ou 2, dans lequel le sujet présente 10 répétitions dans la région polymorphe des deux allèles pour le transporteur de dopamine humain (hDAT).

4. Procédé selon l'une quelconque des revendications 1 à 3 dans lequel le peptide reconnu est **caractérisé par** SEQ ID NO : 2.

5. Procédé selon l'une quelconque des revendications 1 à 3 dans lequel le peptide reconnu est **caractérisé par** SEQ ID NO : 4.

6. Procédé selon l'une quelconque des revendications 1 à 3 dans lequel le peptide reconnu est **caractérisé par** SEQ ID NO : 5.

7. Procédé pour estimer l'efficacité d'un régime de traitement pour le THADA chez un sujet, comprenant l'estimation d'un échantillon d'un liquide biologique obtenu du sujet pour la présence d'anticorps capables de reconnaître au moins un peptide choisi parmi ceux **caractérisés par** SEQ ID NO : 2, 4, 5.

8. Procédé selon la revendication 7, dans lequel une seconde estimation est effectuée au moins quatre semaines après la première estimation.

9. Procédé selon la revendication 7 ou 8, dans lequel le sujet présente 9 répétitions dans la région polymorphe de l'un ou des deux allèles pour le transporteur de dopamine humain (hDAT).

10. Peptide qui est choisi parmi SEQ ID NO : 2, 4, 5.

11. Support destiné à une utilisation avec un test ELISA qui comporte un ou plusieurs peptides choisis parmi SEQ ID NO : 2, 4, 5 adsorbés dessus.

12. Kit comprenant un ou plusieurs peptides **caractérisés par** une séquence choisie parmi SEQ ID NO : 2, 4, 5, chacun lié à un support ou un substrat de test ELISA.


### SEQUENCE LISTING

<110> Instituto Superiore di Sanita
   Pharm-Holding, CJSC
<120> BIOMARKERS
<130> PN810701WO
<160> 7
<170> PatentIn version 3.5
<210> 1
   <211> 19
   <212> PRT
   <213> Homo sapiens
<400> 1
   Ser Thr Ser Pro Ala Glu Glu Phe Tyr Thr Arg His Val Leu Gln Ile
   1               5                   10                  15
   His Arg Ser
<210> 2
   <211> 20
   <212> PRT
   <213> Homo sapiens
<400> 2
   Pro Trp Thr Ser Cys Lys Asn Ser Trp Asn Thr Gly Asn Cys Thr Asn
   1               5                   10                  15
   Tyr Phe Ser Glu
               20
<210> 3
   <211> 17
   <212> PRT
   <213> Homo sapiens
<400> 3
   Ser Thr Ser Pro Ala Glu Glu Phe Tyr Thr Arg Val Leu Gln Ile Arg
   1               5                   10                  15
   Ser
<210> 4
   <211> 19
   <212> PRT
   <213> Homo sapiens
<400> 4
   Pro Trp Ile His Cys Asn Asn Ser Trp Asn Ser Pro Asn Cys Ser Asp
   1               5                   10                  15
   Ala Pro Gly
<210> 5
   <211> 19
   <212> PRT
   <213> Homo sapiens
<220>
   <221> DISULFID
   <222> (5)..(14)
<400> 5
   Pro Trp Ile His Cys Asn Asn Ser Trp Asn Ser Pro Asn Cys Ser Asp
   1               5                   10                  15
   Ala Pro Gly
<210> 6
   <211> 23
   <212> DNA
   <213> Homo sapiens
<400> 6
   tgtggtgtag ggaacggcct gag 23
<210> 7
   <211> 24
   <212> DNA
   <213> Homo sapiens
<400> 7
   cttcctggag gtcacggctc aagg 24